# FASCICULE DE BREVET EUROPEEN

(11) **EP 0 774 662 B1**
(45) Date de publication et mention de la délivrance du brevet: **26.01.2005**
(21) Numéro de dépôt: 96402440.0
(22) Date de dépôt: 14.11.1996
(51) Int. Cl.: G01N 27/403, G01N 33/00

(54) **Procédé de réalisation collective de puces avec des électrodes sélectivement recouvertes par un dépôt**
Serienherstellung von Chips mit beschichteten selektiven Elektroden
Batch manufacturing method for chips having coated selective electrodes

(30) Priorité: 17.11.1995 FR 9513659
(43) Date de publication de la demande: 21.05.1997
(73) Titulaire: COMMISSARIAT A L'ENERGIE ATOMIQUE, 75015 Paris (FR)
(72) Inventeur: Caillat, Patrice, 38130 Echirolles (FR); Nicolas, Gérard, 38340 Voreppe (FR); Teoule, Robert, 38000 Grenoble (FR)
(74) Mandataire: Poulin, Gérard

(56) Documents cités:
- US-A- 4 676 761
- US-A- 5 103 557
- US-A- 5 120 421
- SENSORS AND ACTUATORS A, vol. 43, 1 Janvier 1994, pages 296-301, XP000567518 KAKEROW R ET AL: "A MONOLITHIC SENSOR ARRAY OF INDIVIDUALLY ADDRESSABLE MICROELECTRODES"
- EUROSENSORS VII CONFERENCE AND EXHIBITION, BUDAPEST, HUNGARY, 26-29 SEPT. 1993, vol. B19, no. 1-3, ISSN 0925-4005, SENSORS AND ACTUATORS B (CHEMICAL), APRIL 1994, SWITZERLAND, pages 675-677, XP000449927 FIACCABRINO G C ET AL: "Array of individually addressable microelectrodes"
- PATENT ABSTRACTS OF JAPAN vol. 013, no. 460 (P-946), 18 Octobre 1989 & JP 01 179995 A (FUJITSU LTD), 18 Juillet 1989,
- SENSORS AND ACTUATORS B, vol. B21, no. 1, 1 Juillet 1994, pages 33-37, XP000479669 HERMES T ET AL: "AN AMPEROMETRIC MICROSENSOR ARRAY WITH 1024 INDIVIDUALLY ADDRESSABLE ELEMENTS FOR TWO-DIMENSIONAL CONCENTRATION MAPPING"

## Description

### Domaine technique

La présente invention concerne un procédé de réalisation collective de puces avec des électrodes sélectivement recouvertes par un dépôt.

L'invention trouve des applications notamment pour la fabrication de capteurs ou d'autres éléments sensibles miniaturisés dans lesquels des puces avec un grand nombre d'électrodes doivent être réalisées. Ces électrodes pour être adaptées à leur fonction spécifique dans le capteur ou l'élément sensible doivent être recouvertes de matériaux appropriés.

A titre d'exemple, l'invention s'applique à la réalisation d'éléments miniaturisés tels que des "bio-chips" qui sont des puces comportant une partie de circuit électrique réalisée sur un substrat, tel qu'un champ d'électrodes, et une partie biologique réalisée à la surface de la puce. Dans cet exemple, il convient de déposer sélectivement sur les électrodes des composés chimiques compatibles avec les produits biologiques.

### Etat de la technique antérieure

Pour former sur des électrodes un dépôt, on fait appel actuellement à des procédés connus de microélectronique tels que les procédés de lithographie.

Cette technique permet de traiter sur une même tranche de semi-conducteur simultanément un grand nombre de puces, pour déposer sélectivement un composé chimique sur des électrodes déterminées de chaque puce.

Comme le montre le document (1) dont la référence est indiquée à la fin de la présente description, un procédé de réalisation de dépôts selon la première technique comporte les étapes successives suivantes :
- dépôt sur la tranche de semi-conducteur d'un fond continu conducteur électrique en contact avec les électrodes sur lesquelles on veut former un dépôt,
- formation sur le fond continu d'une couche de résine,
- ouvertures de fenêtres dans la résine au-dessus des électrodes choisies,
- formation électrochimique du matériau de dépôt dans les fenêtres en utilisant le fond continu comme électrode, une contre-électrode indépendante étant placée dans un bain électrochimique avec la tranche,
- élimination de la résine et du fond continu, autour du matériau déposé.

Ce procédé est classique. Il permet de déposer localement sur une tranche de substrat, par exemple sur des électrodes déterminées, un métal ou tout autre composé chimique.

Toutefois, dans certaines applications il est nécessaire de recouvrir différentes électrodes d'une même puce avec des matériaux différents. Ces matériaux sont dédiés à la fonction spécifique de chaque électrode.

Avec le procédé électrochimique mentionné, il est nécessaire pour déposer différents matériaux sur différents sites ou électrodes d'une tranche, de former autant de couches de résine que de matériaux différents à déposer. Il est en effet nécessaire, pour le dépôt de chaque matériau de mettre à nu les régions où le matériau doit être déposé et de protéger les régions où il ne doit pas l'être.

La succession d'un grand nombre d'étapes de dépôt de résine et d'opérations de lithographie pour pratiquer dans chaque couche de résine les ouvertures nécessaires, correspondant aux électrodes devant être revêtues d'un matériau donné, rend le procédé complexe.

Par ailleurs, on connaît, comme illustrée par le document (2), référencé à la fin de la présente description, une technique multiplexant les électrodes d'une puce pour réaliser à partir de cette puce, un capteur à matrice d'électrodes apte à effectuer des mesures de milieux chimiques ou biochimiques.

Un but de la présente invention est de proposer un traitement collectif de puces pour la réalisation de puces avec des électrodes sélectivement recouvertes par un dépôt (les électrodes étant multiplexées lorsque le nombre d'électrodes par puce est important).

Un but de l'invention est aussi de proposer un tel procédé qui ne nécessite pas une succession d'étapes de photolithographie ni une manipulation individuelle de chaque puce.

Un but de l'invention est encore de proposer un procédé simple et fiable qui ne présente pas les inconvénients ou limitations précédemment évoquées.

Un but de l'invention est également de proposer un procédé capable de réaliser avec un rendement de fabrication élevé simultanément un très grand nombre de puces, ayant chacune un nombre important d'électrodes.

### Exposé de l'invention

Pour atteindre les buts mentionnés ci-dessus, l'invention a plus précisément pour objet un procédé de réalisation collective de puces avec des électrodes sélectivement recouvertes par un dépôt, caractérisé en ce que le procédé comporte les étapes suivantes :
a) réalisation sur un support (substrat) d'un ensemble de puces comportant chacune une pluralité d'électrodes,
b) test individuel de validité de chaque puce et formation d'au moins un réseau de connexions électriques, respectivement pour l'adressage d'au moins un ensemble d'électrodes dites appariées, de différentes puces, chaque réseau étant réalisé selon un plan de connexion déterminé et reliant électriquement entre elles des puces valides,
c) réalisation d'un dépôt successivement sur les électrodes de chaque ensemble d'électrodes appariées, respectivement par trempage du support dans un bain électrochimique approprié et par application de tensions de polarisation appropriées sur le réseau de connexion correspondant à l'ensemble d'électrodes appariées pour provoquer sélectivement une réaction électrochimique sur les électrodes de l'ensemble d'électrodes appariées.

Chaque fois qu'un matériau différent doit être déposé sur des électrodes, le support est trempé dans un autre bain électrolytique approprié.

Au sens de la présente invention, on entend par puce valide une puce qui ne présente pas de défaut rédhibitoire par rapport au traitement collectif de l'ensemble des puces.

A titre d'exemple, un défaut rédhibitoire peut être un court-circuit dans l'une des puces qui relierait son alimentation à sa masse. Ce type de défaut pouvant mettre en défaut l'ensemble des puces qui lui sont reliées.

De façon avantageuse, lorsque les dépôts sont effectués, le support (la tranche) comportant les puces peut être découpé pour individualiser les puces, afin de pouvoir les utiliser séparément.

Lors de ce découpage, les liaisons électriques qui formaient le réseau de puces sont automatiquement interrompues.

Les puces retrouvent ainsi leur indépendance électrique et peuvent être utilisées individuellement dans l'application à laquelle elles sont destinées.

Dans une telle mise en oeuvre, on note que la connexion des puces entre-elles n'est qu'une connexion provisoire pour permettre la formation du dépôt sur certaines électrodes. Cette connexion disparaît lors de la découpe du support, et chaque puce retrouve une fonctionnalité propre. Ainsi, le réseau de connexion formé ne constitue pas un objet final mais simplement une étape intermédiaire.

Il convient de noter également que le réseau de connexion n'a pas pour but de créer une fonction commune à laquelle chaque puce valide apporte une contribution, mais sert simplement à effectuer le dépôt sélectif.

L'ordre des étapes du test individuel de validité des puces et la connexion des puces peuvent être différents selon les modes de mise en oeuvre de l'invention.

Selon un premier mode, l'étape b) comporte successivement le test individuel de la validité de chaque puce puis la connexion des puces valides entre elles.

Dans ce mode de mise en oeuvre de l'invention, seules les puces valides sont reliées dans le réseau, ce qui permet de former directement un réseau fonctionnel.

Selon un autre mode, l'étape b) comporte successivement :
- le test individuel de validité de chaque puce,
- la connexion des puces entre elles selon le plan de connexion,
- déconnexion des puces non valides.

La déconnexion des puces non valides peut être obtenue par sciage ou par tir laser.

En effet, selon un aspect de l'invention, la connexion des puces peut être réalisée par des liaisons électriques comportant des points dits de fusion, aptes à être fondus sous l'effet d'un faisceau laser pour interrompre les liaisons. La déconnexion est alors réalisée par l'application d'un faisceau laser sur les points de fusion des liaisons électriques reliant les puces non valides au réseau de puces.

Selon que les puces présentent un nombre plus ou moins grand d'électrodes, la réalisation du ou des réseaux de connexions peut être différente.

Une première possibilité consiste à former sur le support un ensemble de bornes dites bornes collectives d'adressage et à relier respectivement dans chaque réseau de connexions électriques une borne collective d'adressage aux électrodes d'un ensemble d'électrodes appariées.

Ainsi, une tension appliquée sur l'une des bornes collectives est transmise sur différentes puces à chacune des électrodes de l'ensemble d'électrodes appariées correspondant.

Une autre possibilité consiste à former sur chaque puce des bornes dites bornes individuelles d'adressage et sur le support un ensemble de bornes collectives d'adressage. Les bornes individuelles d'adressage de chaque puce permettent, par exemple, d'appliquer à un circuit de multiplexage de la puce une adresse de sélection d'une ou de plusieurs électrodes de la puce qui doivent être portées à un potentiel approprié à une réaction électrochimique. Un tel dispositif est bien adapté pour les puces ayant un grand nombre d'électrodes. Pour la réalisation du multiplexage, on peut se reporter au document (3) référencé à la fin de la présente description. A titre indicatif pour une puce avec 2ⁿ électrodes, par exemple 2⁷=128 électrodes, seules n bornes individuelles d'adressage sont nécessaires dans une structure multiplexée de type parallèle. Dans une structure multiplexée de type série, moins rapide néanmoins, une seule borne suffit.

Lors de la formation du réseau de connexions, on relie alors électriquement chaque borne d'adressage individuelle de chaque puce valide, respectivement à une borne collective correspondante, selon un plan de connexion prédéterminé.

Que les bornes collectives soient reliées directement aux électrodes ou à des bornes individuelles d'adressage, le plan de connexion est établi de préférence de manière telle que la déconnexion d'une puce invalide dans le réseau de connexions n'isole pas une puce valide.

A titre d'exemple, les bornes individuelles ou les électrodes peuvent être reliées en parallèle et/ou en série sur les bornes collective.

Le plan de connexion peut aussi comporter une redondance de connexion pour garantir la connexion de toutes les puces valides même dans le cas où des puces non valides sont éliminées du réseau.

Selon un aspect de l'invention, la formation du réseau de connexions peut comporter :
- le dépôt, sur l'ensemble du substrat comportant les puces, d'une couche de matériau conducteur électrique recouverte par une couche de résine photosensible,
- l'insolation de la résine dans des champs correspondant respectivement à des puces valides, les champs correspondant à des puces valides voisines présentant des régions marginales de recouvrement mutuel dans lesquelles sont prévus des motifs correspondant à des bandes conductrices d'interconnexion de lignes de connexion des puces,
- le développement de la résine et la gravure de la couche de matériau conducteur selon les motifs pour former des bandes d'interconnexion.

L'étape de réalisation des dépôts sur les électrodes peut être réalisée au moins de deux façon différentes.

Selon une première mise en oeuvre, on forme le dépôt sur les électrodes de chaque ensemble d'électrodes appariées directement par la réaction électrochimique.

Selon une variante, pour chaque ensemble d'électrodes appariées, avant de provoquer la réaction électrochimique, on forme sur toutes les électrodes une couche d'un composé chimique et on élimine, par la réaction électrochimique le composé chimique sur toutes les électrodes à l'exception des électrodes de l'ensemble d'électrodes appariées adressées.

Selon un aspect de l'invention, il est possible, avant la réalisation des dépôts de l'étape c), de former, au moins sur certaines électrodes, une couche de matériau photosensible. Lors de la réalisation des dépôts de l'étape c) les électrodes sont alors soumises à un rayonnement lumineux, par exemple ultraviolet, qui active la réaction électrochimique sur les électrodes appariées qui sont adressées.

Selon un autre aspect de l'invention, le dépôt réalisé sur les électrodes peut être un dépôt de molécules actives choisies dans le groupe de molécules biochimiques ou biologiques. Le dépôt peut également être un dépôt métallique ou de polymère.

D'autres caractéristiques et avantage de la présente invention ressortiront mieux de la description qui va suivre, en référence aux figures des dessins annexés, donnée à titre purement illustratif et non limitatif.

### Brève description des figures

- la figure 1 montre un exemple de réalisation collective de puces reliées dans un réseau de connexions,
- la figure 2 montre un autre exemple de réalisation collective de puces reliées dans un réseau de connexions,
- la figure 3 est une vue à échelle agrandie de puces et montre un exemple d'interconnexion de puces.
- les figures 4A et 4B montrent des champs de photolithographie correspondant à des étapes successives de la réalisation collective de puces, conformément à l'invention.

### Description détaillée de modes de mise en oeuvre de l'invention

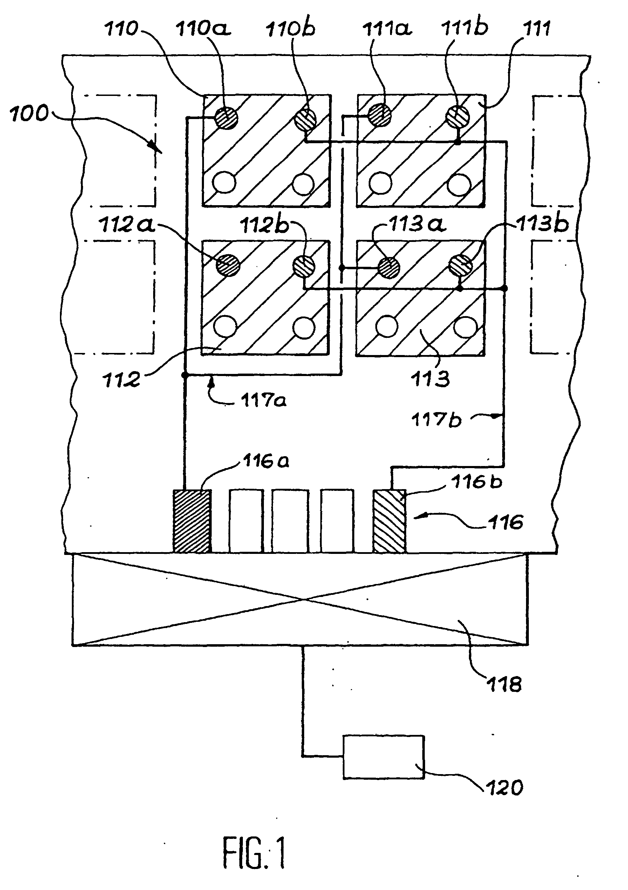

La figure 1 montre un substrat de support 100 sur lequel on a réalisé une pluralité de puces électroniques 110, 111, 112, 113. Les puces sont réalisées selon les procédés usuels de microélectronique.

Sur la figure, pour des raisons de lisibilité, seules quatre puces 110, 111, 112, 113 sont représentées, toutefois, la même tranche de substrat peut comporter un grand nombre de puces, par exemple de 20 à 2000.

Les puces 110, 111, 112, 113 dans l'exemple illustré, sont identiques les unes aux autres. Elles comportent chacune un nombre limité d'électrodes. Afin de les distinguer, les électrodes des puces portent la même référence que la puce suivie respectivement d'une lettre a, b.

Le substrat 100 comporte également des bornes d'adressage 116, 116a, 116b reliées à un connecteur 118. Les bornes 116, 116a, 116b sont réalisées de préférence à la périphérie du substrat 100. Le connecteur 118 permet à partir d'une source de tension 120 représentée très schématiquement d'appliquer aux bornes 116 des tensions convenables pour initier sélectivement sur certaines électrodes des dépôts électrochimiques. Une autre borne de la source de tension est reliée à une contre-électrode immergée dans une solution électrochimique appropriée.

Comme le montre la figure 1, on définit sur la plaque de substrat, des ensembles d'électrodes appariées. Les électrodes appariées, qui sont situées sur différentes puces sont reliées respectivement à une borne d'adressage 116 encore appelée borne collective d'adressage.

Ainsi, à titre d'exemple, on relie à la borne 116a les électrodes 110a, 111a, 112a, 113a qui forment un premier ensemble d'électrodes appariées. Les électrodes 110b, 111b, 112b, 113b forment un deuxième ensemble d'électrodes appariées. Les réseaux de connexions, c'est-à-dire les liaisons électriques qui relient respectivement les électrodes des premier et deuxième ensembles d'électrodes appariées, entre elles et aux bornes 116a et 116b, portent respectivement les références 117a et 117b.

Des liaisons électriques non représentées relient de la même façon d'autres électrodes appariées entre elles, et à une des bornes d'adressage. Les puces sont donc électriquement reliées dans des réseaux de connexions.

Dans l'exemple de la figure, les liaisons électriques sont directement raccordées aux électrodes. Un test individuel des puces permet de distinguer les puces valides des puces invalides. Les électrodes des puces invalides ne sont pas reliées aux bornes 116.

L'ensemble de la tranche 100 équipée des puces 110, 111, 112, 113 est trempée dans un bain électrochimique correspondant à un composé chimique à déposer sur les électrodes de l'un des ensembles d'électrodes appariées.

Une tension convenable délivrée par la source de tension est appliquée par l'intermédiaire du connecteur 118 à une borne collective, par exemple 116a, qui correspond à l'ensemble d'électrodes appariées choisi 110a, 111a, 112a, 113a.

La tension transmise aux électrodes concernées, par le réseau d'interconnexions 117a, provoque pour ces électrodes le dépôt du composé chimique.

La tranche de substrat peut être ensuite rincée puis successivement trempée dans autant de bains électrochimiques que nécessaire pour déposer différents composés chimiques sur des électrodes de différents ensembles d'électrodes appariés, en appliquant sur les bornes 116 les tensions appropriées.

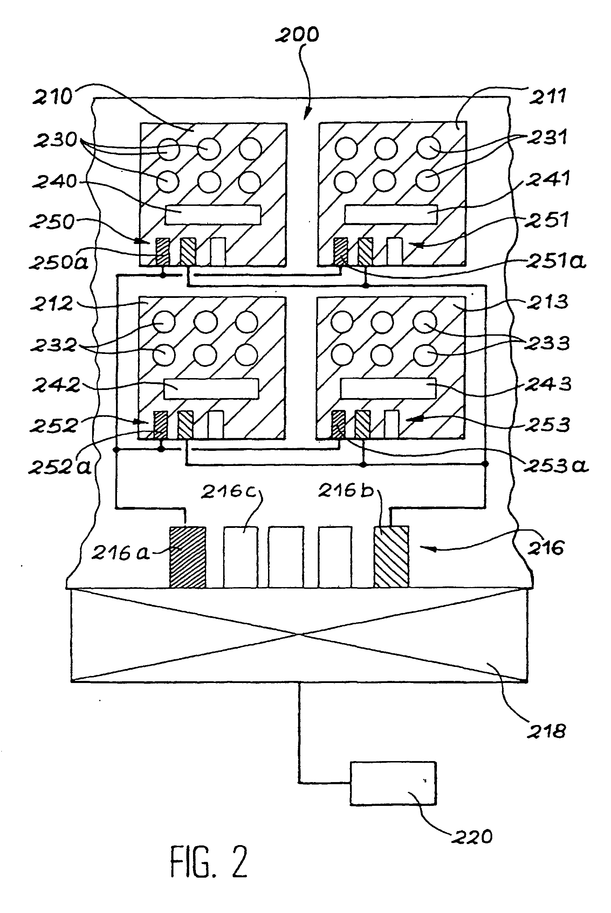

La figure 2 donne un autre exemple de réalisation d'une tranche de substrat équipée d'une pluralité de puces. Cet exemple concerne des puces qui comportent plus particulièrement un grand nombre d'électrodes.

Des références identiques auxquelles on a ajouté 100 sont attribuées à des éléments identiques ou similaires à ceux de la figure 1.

On retrouve ainsi sur la figure 2, un substrat 200 sur lequel est réalisée une pluralité de puces 210, 211, 212, 213. Seules quelques puces sont représentées dans un souci de clarté de la figure.

Chaque puce comporte une pluralité d'électrodes 230, 231, 232, 233, un circuit de multiplexage 240, 241, 242, 243 et un ensemble de bornes individuelles d'adressage 250, 251, 252, 253.

Le substrat 200 comporte aussi des bornes collectives d'adressage 216. Un connecteur 218 permet de relier les bornes 216 à une source de tension extérieure 220 apte à délivrer des signaux d'adressage. La source de tension extérieure peut être une interface spécialement adaptée d'un micro-ordinateur, par exemple.

Après un test de validité des puces celles-ci sont reliées entre elles dans des réseaux de puces. Les réseaux sont établis selon des plans de connexion dans lesquels des bornes d'adressage individuelles équivalentes de chaque puce sont respectivement reliées à des bornes collectives d'adressage correspondantes du support.

A titre d'illustration, les bornes individuelles 250a, 251a, 252a et 253a des puces 210, 211, 212, 213 sont toutes reliées entre elles et reliées à une borne collective 216a correspondante.

Le circuit de multiplexage interne 240, 241, 242, 243 de chaque puce permet d'adresser sélectivement une ou plusieurs électrodes sélectionnées, en fonction d'un signal d'adressage qui est appliqué par exemple sur l'une des bornes individuelles de la puce.

A titre d'exemple sur une première borne d'adressage 250a, 251a, 252a, 253a de chaque puce sont appliqués les signaux d'adressage des électrodes provenant de la borne collective 216a.

Des deuxièmes bornes individuelles de chaque puce, reliées par exemple à la borne collective 216b, sont destinées à l'alimentation électrique des puces.

Des troisièmes bornes individuelles de chaque puce, sont reliées par exemple à la borne collective 216c par des liaisons non représentées sur la figure 2. Ces bornes sont destinées à fournir à chaque puce une tension de travail, c'est-à-dire une tension appropriée à la réaction chimique envisagée, cette tension étant distribuée sur la ou les électrodes adressées par le circuit de multiplexage interne de chaque puce.

Enfin, des quatrièmes bornes individuelles (non représentées) des puces peuvent être reliées à une borne collective du substrat pour une remise à zéro logique des puces.

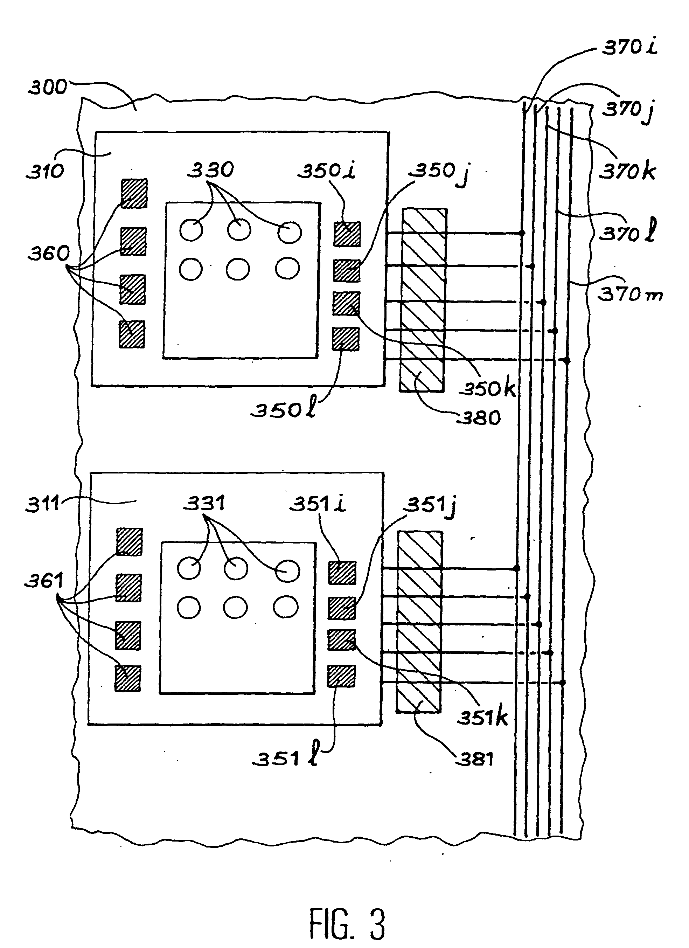

La figure 3 montre à plus grande échelle un exemple d'interconnexion de puces. Deux puces 310, 311, sensiblement identiques comportent chacune un jeu d'électrodes 330, 331 et un ensemble de bornes individuelles d'adressage référencées 350i, 350j, 350k, 350l, 351i, 351j, 351k, 351l.

Chaque puce comporte également un ensemble 360, 361 de plots de test individuels. Ces plots sont prévus pour coopérer avec une carte de test à pointes d'un type classique.

Sur le substrat 300, au voisinage des puces, sont prévues des lignes de connexion électrique 370i, 370j, 370k, 370l qui sont respectivement reliées avec les bornes individuelles 350i, 350j, 350k, 350l de la puce 310, et avec les bornes individuelles 351i, 351j, 351k, 351l de la puce 311. Les bornes individuelles des puces sont reliées de préférence en parallèle à ces lignes de connexion pour permettre l'isolation des puces non valides sans affecter le fonctionnement des puces valides environnantes.

Le substrat comporte également des zones 380, 381 dites de reconfiguration associées respectivement à chaque puce. Dans ces zones les lignes électriques de connexion qui relient les bornes de connexion aux lignes 370i, 370j, 370k, 370l, peuvent être fondues.

Selon un mode de mise en oeuvre de l'invention, toutes les puces sont initialement interconnectées selon le plan d'interconnexion décrit précédemment. Les puces sont ensuite testées en appliquant et en mesurant sur des plots 360, 361 des tensions de test. Ce test permet de distinguer les puces valides de celles qui présentent un défaut tel qu'un court-circuit.

Les puces non valides sont isolées du réseau de connexion en effectuant dans la zone de reconfiguration correspondante un tir laser pour fondre des points de fusion des lignes de connexion dans cette zone, et interrompre ces lignes.

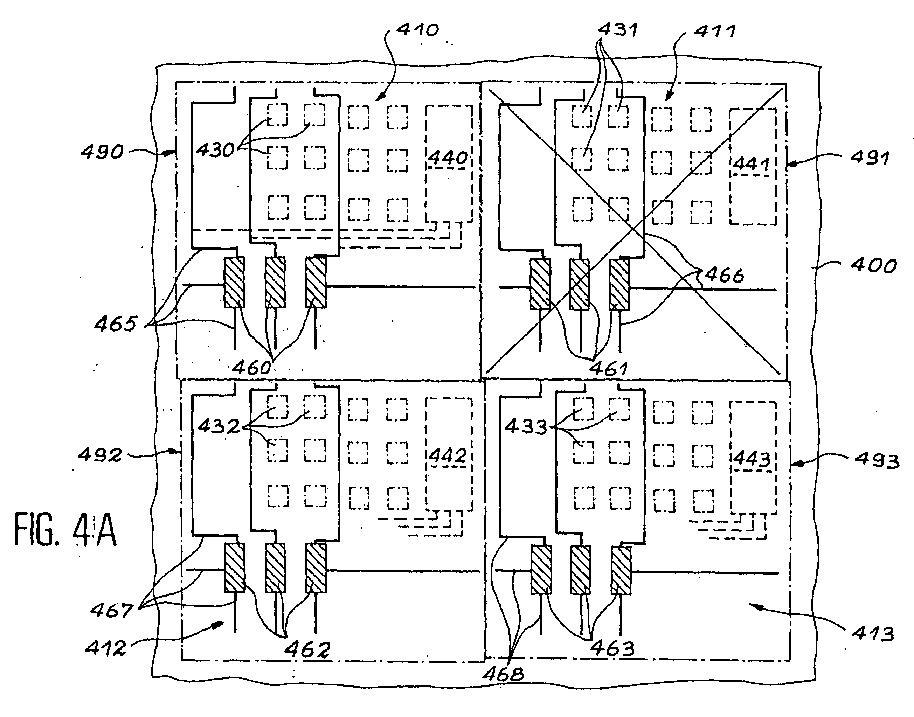

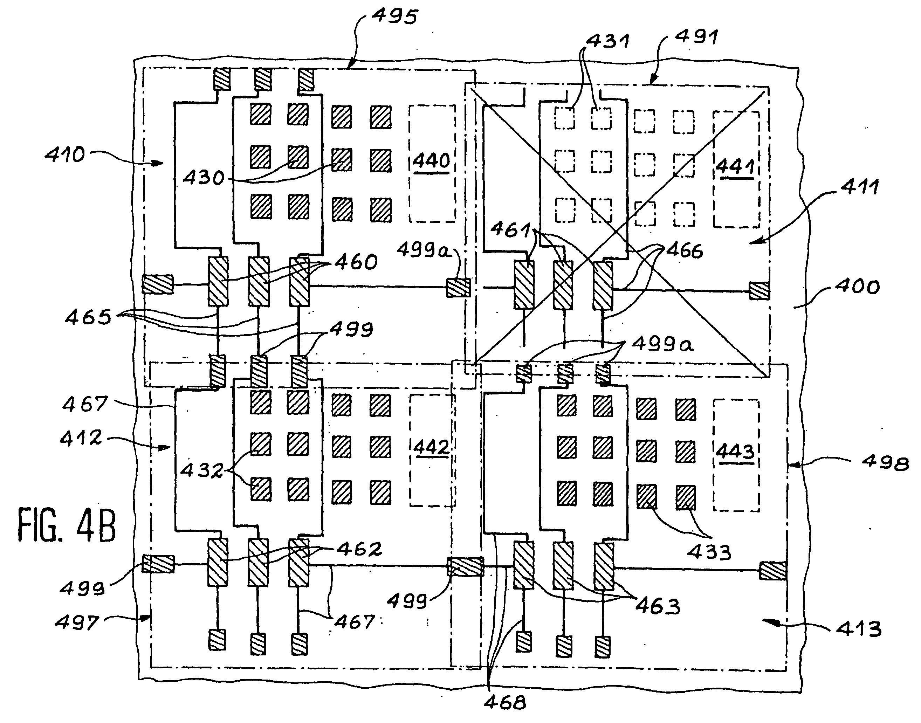

Les figures 4A et 4B illustrent la réalisation collective de puces sur un substrat.

La figure 4A correspond à une étape de photolithographie et montre à échelle agrandie une portion d'un substrat 400 dans lequel sont réalisées des puces 410, 411, 412, 413 sensiblement identiques.

Les puces comportent différents éléments tels qu'un circuit de multiplexage 440, 441, 442, 443, des électrodes 430, 431, 432, 433 et des plots de test 460, 461, 462, 463, par exemple.

Les différents éléments sont réalisés au cours de différentes étapes de lithographie correspondant à différents niveaux conducteurs superposés. Lors de ces étapes de lithographie on dépose sur la tranche de substrat une couche de matériau conducteur et une couche de résine. La résine est insolée à travers un masque correspondant à des motifs que l'on souhaite réaliser dans la couche conductrice. Après le développement de la résine, la couche de matériau conducteur est gravée pour éliminer les parties non protégées par la résine.

En raison de la taille relativement importante du substrat, l'ensemble de la couche de résine n'est pas simultanément insolée.

On insole successivement des portions de la couche de résine, à travers un même masque qui est déplacé par rapport au substrat dans un appareil usuellement désigné par "steppeur" ou "photorépéteur".

Ces portions sont désignées par le terme "champ" et correspondent par exemple respectivement à l'emplacement d'une puce sur le substrat.

Sur la figure 4A, sont respectivement représentés quatre champs 490, 491, 492, 493 qui correspondent aux puces 410, 411, 412, 413.

On constate que les champs sont juxtaposés. Dans les champs de la figure 4A sont réalisés des motifs conducteurs correspondant aux plots de test 460, 461, 462, 463 et à des lignes de connexion 465, 466, 467, 468 s'étendant depuis ces plots jusqu'en des régions marginales des champs.

La figure 4A correspond à un avant-dernier niveau conducteur.

Dans ce niveau conducteur les extrémités des lignes de connexion ne sont pas reliées et les puces sont électriquement isolées.

On peut noter que les circuits de multiplexage 440, 441, 442, 443 qui sont réalisés antérieurement et les emplacements des électrodes 411, 411, 412 et 413 qui sont réalisés postérieurement à l'étape de lithographie de la figure 4A, sont représentés en lignes discontinues.

Lorsque les plots de test sont achevés, chaque puce est individuellement testée afin de déterminer sa validité.

La figure 4B correspond à une dernière étape de lithographie pour la mise en forme d'un dernier niveau conducteur.

Cette étape est réalisée dans des deuxièmes champs 495, 497 et 498 qui correspondent aux puces 410, 412, 413 que le test individuel a révélées valides.

Les deuxièmes champs d'insolation 495, 497 et 498 sont plus grands que les premiers champs 490, 492, 493 correspondants auxquels ils sont sensiblement superposés. Les champs 495 et 497 d'une part et les champs 497 et 498 d'autre part, présentent respectivement des régions marginales de recouvrement. Dans ces régions sont prévus des motifs 499 qui correspondent à des bandes conductrices d'interconnexion reliant entre elles des lignes de connexion en regard 465 et 467, et 467 et 468.

La partie du substrat correspondant au champ 491 n'est pas insolée lors de la dernière étape de lithographie. Le matériau conducteur du dernier niveau conducteur y est donc totalement éliminé et la puce 411, jugés non valide lors du test individuel, reste isolée.

Des parties de motif 499a sont formées sur les bords périphériques des champs 495 et 498 tournés vers le champ 491. Toutefois, il n'y a pas de partie de motif correspondant sur le champ 491, ni de recouvrement suffisant pour interconnecter les lignes 466 de la puce 411 avec les lignes 465 et 468 en regard des puces 410 et 413.

L'isolation électrique de la puce 411 n'est pas préjudiciable à la connexion des puces voisines dans le réseau de connexions en raison d'une redondance dans les lignes de connexion.

On obtient finalement un ou plusieurs réseaux auxquels seules les puces valides sont connectées.

Chaque réseau est relié à une ou plusieurs bornes d'adressage collectif formées sur le substrat pour appliquer aux puces des tensions convenables afin d'initier les réactions électrochimiques. Ces bornes ne sont pas représentées sur les figures 4A et 4B.

### LISTE DES DOCUMENTS CITES DANS LA PRESENTE DEMANDE

***(1)***
   "Fluxless Flip-Chip Technology" de P. Caillat et G. Nicolas.
***(2)***
   "A monolithic sensor array of individually addressable microelectrodes" de R. Kakerow et al. dans "Sensors and Actuators" A, 43 (1994) pp. 296-301
***(3)***
   "Array of individually addressable microelectrodes" de G. C Fiaccabrino et al., dans "Sensors and Actuators" B, 18-19 (1994), pp. 675-677

## Revendications

1. Procédé de réalisation collective de puces comportant des électrodes sélectivement recouvertes par un dépôt, **caractérisé en ce que** le procédé comporte les étapes suivantes :
a) réalisation sur un support (100, 200, 300,400) d'un ensemble de puces (110, 210, 310, 410, 111, 211, 311, 411, 112, 212, 412, 113, 213, 413) comportant chacune une pluralité d'électrodes (110a, 110b, 111a, 111b, 230, 231, 232, 233, 330, 331, 430, 431, 432, 433),
b) test individuel de validité de chaque puce et formation d'au moins un réseau de connexions (117a, 117b, 370i, 370j, 370k, 370l, 465, 466, 467, 468, 499) électriques, respectivement pour l'adressage d'au moins un ensemble d'électrodes dites appariées, de différentes puces, chaque réseau étant réalisé selon un plan de connexion déterminé et reliant électriquement entre elles des puces valides,
c) réalisation d'un dépôt successivement sur les électrodes de chaque ensemble d'électrodes appariées, respectivement par trempage du support dans un bain électrochimique approprié et par application de tensions de polarisation appropriées sur le réseau de connexions correspondant à l'ensemble d'électrodes appariées pour provoquer sélectivement une réaction électrochimique sur les électrodes de l'ensemble d'électrodes appariées.

2. Procédé selon la revendication 1, **caractérisé en ce que** l'étape b) comporte successivement le test individuel de la validité de chaque puce puis la connexion des puces valides entre elles.

3. Procédé selon la revendication 1, **caractérisé en ce que** l'étape b) comporte successivement :
- le test individuel de validité de chaque puce,
- la connexion des puces entre elles selon le plan de connexion,
- déconnexion des puces non valides.

4. Procédé selon la revendication 3, **caractérisé en ce que** la connexion des puces est réalisée par des liaisons électriques comportant des points dits de fusion (380), aptes à être fondus sous l'effet d'un faisceau laser pour interrompre les liaisons, et la déconnexion étant réalisée par l'application d'un faisceau laser sur les points de fusion des liaisons électriques reliant les puces non valide au réseau de puces.

5. Procédé selon l'une quelconque des revendications précédentes, **caractérisé en ce que** l'on forme sur le support (100, 200) un ensemble de bornes (116, 216) dites bornes collectives d'adressage et on relie respectivement dans chaque réseau de connexions électriques une borne collective d'adressage aux électrodes d'un ensemble d'électrodes appariées.

6. Procédé selon l'une quelconque des revendications 1 à 4, **caractérisé en ce que** l'on réalise sur chaque puce des bornes dites bornes individuelles (250, 251, 252, 253) d'adressage des électrodes de la puce, on réalise sur le support un ensemble de bornes dites bornes collectives d'adressage (216, 216a, 216b), et lors de la formation du réseau de connexions, on relie électriquement chaque borne d'adressage individuelle de chaque puce valide, respectivement à une borne collective correspondante, selon le plan de connexion.

7. Procédé selon la revendication 1, **caractérisé en ce qu'**avant l'étape c) on forme au moins sur certaines électrodes une couche de matériau photosensible, et lors de l'étape c) on soumet les électrodes à un rayonnement lumineux pour activer la réaction électrochimique.

8. Procédé selon la revendication 1, **caractérisé en ce qu'**on forme le dépôt sur les électrodes de chaque ensemble d'électrodes appariées directement par la réaction électrochimique.

9. Procédé selon la revendication 1, **caractérisé en ce que** pour chaque ensemble d'électrodes appariées, avant de provoquer la réaction électrochimique, on forme sur toutes les électrodes une couche d'un composé chimique et on élimine, par la réaction électrochimique le composé chimique sur toutes les électrodes à l'exception des électrodes de l'ensemble d'électrodes appariées adressées.

10. Procédé selon la revendication 1, **caractérisé en ce qu'**après la réalisation des dépôts sur chaque ensemble d'électrodes on découpe le support pour séparer les puces les unes des autres.

11. Procédé selon la revendication 1, **caractérisé en ce que** les puces sont sensiblement identiques et chaque ensemble d'électrodes appariées comporte une électrode de chaque puce.

12. Procédé selon l'une quelconque des revendications précédentes, **caractérisé en ce que** le dépôt réalisé sur les électrodes est un dépôt de molécules actives choisies dans le groupe de molécules biochimiques ou biologiques.

13. Procédé selon l'une quelconque des revendications précédentes, **caractérisé en ce que** la formation du réseau de connexions comporte :
- le dépôt, sur l'ensemble du substrat comportant les puces, d'une couche de matériau conducteur électrique recouverte par une couche de résine photosensible,
- l'insolation de la résine dans des champs (495, 497, 498) correspondant respectivement à des puces valides, les champs correspondant à des puces valides voisines présentant des régions marginales de recouvrement mutuel dans lesquelles sont prévus des motifs (499) correspondant à des bandes conductrices d'interconnexion de lignes de connexion des puces,
- le développement de la résine et la gravure de la couche de matériau conducteur selon les motifs pour former des bandes d'interconnexion.

## Patentansprüche

1. Verfahren zur Serienherstellung von Chips mit selektiv beschichteten Elektroden,
**dadurch gekennzeichnet, dass** das Verfahren die folgenden Schritte umfasst:
a) die Herstellung einer Gruppe von Chips (110, 210, 310, 410, 111, 211, 311, 411, 112, 212, 412, 113, 213, 413), jeder eine Vielzahl Elektroden (110a, 110b, 111a, 111b, 230, 231, 232, 233, 330, 331, 430, 431, 432, 433) umfassend, auf einem Träger (100, 200, 300, 400),
b) einen individuellen Freigabetest jedes Chips und Ausbildung wenigstens eines elektrischen Verbindungsnetzwerks (117a, 117b, 370i, 370j, 370k, 370l, 465, 466, 467, 468, 499), jeweils zur Adressierung wenigstens einer Gruppe sogenannter gepaarter Elektroden verschiedener Chips, wobei jedes Netzwerk nach einem bestimmten Verbindungsplan realisiert wird und freigegebene Chips miteinander verbindet,
c) die sukzessive Realisierung einer Beschichtung der Elektroden jeder Gruppe gepaarter Elektroden, jeweils durch das Eintauchen des Trägers in ein entsprechendes elektrochemisches Bad und durch das Anlegen entsprechender Polungsspannungen an das der Gruppe gepaarter Elektroden entsprechende Verbindungsnetzwerk, um bei den Elektroden der Gruppe gepaarter Elektroden selektiv eine elektrochemische Reaktion zu bewirken.

2. Verfahren nach Anspruch 1, **dadurch gekennzeichnet, dass** der Schritt b) sukzessive den individuellen Freigabetest jedes Chips und dann die Verbindung der freigegebenen Chips miteinander umfasst.

3. Verfahren nach Anspruch 1, **dadurch gekennzeichnet, dass** der Schritt b) sukzessive umfasst:
- den individuellen Freigabetest jedes Chips,
- die Verbindung der Chips miteinander gemäß dem Verbindungsplan,
- die Unterbrechung der Verbindung nicht freigegebener Chips.

4. Verfahren nach Anspruch 3, **dadurch gekennzeichnet, dass** die Verbindung der Chips durch elektrische Verbindungen realisiert wird, die sogenannte Schmelzpunkte (380) umfassen, die man mittels Laserstrahl schmelzen kann, um die Verbindungen zu unterbrechen, und dass die Unterbrechung der elektrischen Verbindungen, welche die nicht freigegebenen Chips mit dem Chip-Netzwerk verbinden, mittels eines auf die entsprechenden Schmelzpunkte gerichteten Laserstrahls realisiert wird.

5. Verfahren nach einem der vorangehenden Ansprüche, **dadurch gekennzeichnet, dass** man auf dem Träger (100, 200) eine Gruppe von Anschlüssen (116, 216) bildet, Sammeladressierungsanschlüsse genannt, und man in jedem elektrischen Verbindungsnetzwerk jeweils einen Sammeladressierungsanschluss mit den Elektroden einer Gruppe gepaarter Elektroden verbindet.

6. Verfahren nach einem der Ansprüche 1 bis 4, **dadurch gekennzeichnet, dass** man auf jedem Chip Einzelanschlüsse genannte Anschlüsse (250, 251, 252, 253) zur Adressierung der Elektroden des Chips realisiert, man auf dem Träger eine Gruppe von Anschlüssen (216, 216a, 216b), Adressierungssammelanschlüsse genannt, realisiert, und man bei der Bildung des Verbindungsnetzwerks jeden Einzeladressierungsanschluss jedes freigegebenen Chips jeweils mit einem entsprechenden Sammelanschluss verbindet, gemäß dem Verbindungsplan.

7. Verfahren nach Anspruch 1, **dadurch gekennzeichnet, dass** man vor dem Schritt c) wenigstens auf bestimmten Elektroden eine fotosensible Schicht abscheidet und während des Schritts c) die Elektroden einer Lichtstrahlung aussetzt, um die elektrochemische Reaktion zu aktivieren.

8. Verfahren nach Anspruch 1, **dadurch gekennzeichnet, dass** man die Abscheidung auf den Elektroden jeder Gruppe gepaarter Elektroden direkt durch die elektrochemische Reaktion bildet.

9. Verfahren nach Anspruch 1, **dadurch gekennzeichnet, dass** man in jeder Gruppe gepaarter Elektroden, ehe man die elektrochemische Reaktion einleitet, auf allen Elektroden eine Schicht aus einer chemischen Verbindung bildet und durch die elektrochemische Reaktion auf allen Elektroden eliminiert, mit Ausnahme der Elektroden der Gruppe adressierter gepaarter Elektroden.

10. Verfahren nach Anspruch 1, **dadurch gekennzeichnet, dass** man nach der Realisierung der Abscheidungen auf jeder Elektrodengruppe den Träger zerschneidet, um die Chips voneinander zu trennen.

11. Verfahren nach Anspruch 1, **dadurch gekennzeichnet, dass** die Chips im Wesentlichen identisch sind und jede Gruppe gepaarter Elektroden eine Elektrode jedes Chips umfasst.

12. Verfahren nach einem der vorangehenden Ansprüche, **dadurch gekennzeichnet, dass** die auf den Elektroden realisierte Abscheidung eine Abscheidung aktiver Moleküle ist, ausgewählt aus der Gruppe der biochemischen oder biologischen Moleküle.

13. Verfahren nach einem der vorangehenden Ansprüche, **dadurch gekennzeichnet, dass** die Bildung des Verbindungsnetzwerks umfasst:
- die Abscheidung einer Schicht aus elektrisch leitfähigem Material, bedeckt durch eine Fotoresistschicht, auf dem gesamten die Chips umfassenden Substrat,
- die Bestrahlung des Resists in den Feldern (495, 497, 498), die freigegebenen Chips entsprechen, wobei die benachbarten freigegebenen Chips entsprechenden Felder Bereiche gegenseitiger Überlappung darstellen, in denen Muster (499) vorgesehen sind, die leitfähigen Zusammenschaltungsstreifen von Verbindungsleitungen der Chips entsprechen,
- die Entwicklung des Resists und die Ätzung der Schicht aus leitfähigem Material gemäß den Mustem zur Bildung der Zusammenschaltungsstreifen.

## Claims

1. Process for collective manufacture of chips comprising electrodes selectively covered by a deposit, **characterized in that** the process comprises the following steps:
a) make a set of chips (110, 210, 310, 410, 111, 211, 311, 411, 112, 212, 412, 113, 213, 413) on a substrate (100, 200, 300, 400), each comprising several electrodes (110a, 110b, 111a, 111b, 230, 231, 232, 233, 330, 331, 430, 431, 432, 433),
b) individual validity test on each chip, and the formation of at least one network of electrical connections (117a, 117b, 370i, 370j, 370k, 370l, 465, 466, 467, 468, 499) for addressing at least one set of "matched" electrodes of different chips, each network being made in accordance with a given connections plan and electrically connecting valid chips to each other,
c) make a deposit on electrodes in each set of matched electrodes in sequence, by dipping the substrate in an appropriate electrochemical bath and by applying appropriate polarization voltages on the connections network corresponding to the set of matched electrodes, to selectively provoke an electrochemical reaction on the electrodes in the set of matched electrodes.

2. Process according to claim 1, **characterized in that** step b) comprises the individual test on the validity of each chip, then the connection of valid chips to each other, in sequence.

3. Process according to claim 1, **characterized in that** step b) comprises the following in sequence:
- individual validity test on each chip,
- connection of chips to each other in accordance with the connection plan,
- disconnection of invalid chips.

4. Process according to claim 3, **characterized in that** chips are connected by electrical connections including "fuse" points (380), which can be melted under the effect of a laser beam to cut the links, the disconnection being done by applying a laser beam to fuse points of electrical connections connecting invalid chips to the chips network.

5. Process according to any one of the preceding claims, **characterized in that** a set of terminals (116, 216) called collective addressable terminals are formed on the substrate (100, 200), and a collective addressable terminal is connected to a set of matched electrodes in each network of electrical connections.

6. Process according to any one of the claims 1 to 4, **characterized in that** terminals called individual addressable terminals (250, 251, 252, 253) for chip electrodes are made on each chip, and a set of terminals called collective addressable terminals (216, 216a, 216b) are made on the substrate, and when the connection network is being formed, each individual addressable terminal for each valid chip is electrically connected to the corresponding collective terminal, in accordance with the connection plan.

7. Process according to claim 1, **characterized in that** a layer of photosensitive material is formed before step c) on at least some electrodes, and that a light radiation is applied to the electrodes in step c), to activate the electrochemical reaction.

8. Process according to claim 1, **characterized in that** the deposit is formed directly on the electrodes in each set of matched electrodes by the electrochemical reaction.

9. Process according to claim 1, **characterized in that** before provoking the electrochemical reaction for each set of matched electrodes, a layer of a chemical compound is formed on all electrodes, and the chemical compound is eliminated by the electrochemical reaction on all electrodes except for the electrodes in the set of matched addressed electrodes.

10. Process according to claim 1, **characterized in that** after making deposits on each set of electrodes, the support is cut out to separate chips from each other.

11. Process according to claim 1, **characterized in that** the chips are approximately identical and each set of matched electrodes comprises an electrode of each chip.

12. Process according to any one of the preceding claims, **characterized in that** the deposit made on the electrodes is a set of active molecules chosen from the group of biochemical or biological molecules.

13. Process according to any one of the preceding claims, **characterized in that** formation of the connections network includes:
- deposit of a layer of electrical conducting material covered by a layer of photosensitive resin, over the entire substrate containing the chips,
- insolation of the resin in fields (495, 497, 498) corresponding to valid chips, and fields corresponding to adjacent valid chips with marginal regions of mutual coverage in which patterns (499) are provided corresponding to chip connection lines interconnecting conducting strips,
- development of the resin and etching the layer of conducting material according to the patterns to form interconnecting strips.
